# EUROPÄISCHE PATENTSCHRIFT

(11) **EP 0 630 968 B1**
(45) Veröffentlichungstag und Bekanntmachung des Hinweises auf die Patenterteilung: **05.06.2002**
(21) Anmeldenummer: 94108620.9
(22) Anmeldetag: 06.06.1994
(51) Int. Cl.: C12N 15/64, C12N 15/62

(54) **Genetische Selektion von zur Ligandenbindung befähigten Proteinen mittels Signaltransduktion in Mikroorganismen**
Genetic selection of proteins able to bind a ligand by signal-transduction in a microorganism
Selection génétique de proteines capables de fixer un ligand par transduction d'un signal dans des microorganismes

(30) Priorität: 10.06.1993 DE 4319296
(43) Veröffentlichungstag der Anmeldung: 28.12.1994
(73) Patentinhaber: HOECHST AKTIENGESELLSCHAFT, 65926 Frankfurt am Main (DE)
(72) Erfinder: Fritz, Hans-Joachim, D-37075 Göttingen (DE); Hennecke, Frank, D-37075 Göttingen (DE); Kolmar, Harald, D-37077 Göttingen (DE)

(56) Entgegenhaltungen:
- BIOL. CHEM. HOPPE-SEYLER, Bd.374, Nr.9, 1993 Seite F109 HENNECKE F. ET AL. 'A transcriptional signal derived from the dimerisation of immunoglobin fragments located in the periplasmic space of E. coli'
- CELL, Bd.64, 1991 Seiten 891 - 901 B.A. IRVING ET AL. 'The cytoplasmic domain of the cell receptor...'
- CELL, Bd.68, 1992 Seiten 83 - 95 A-M. K. WEGENER ET AL. 'The T cell receptor/CD3 complex is composed of at least two autonomous transduction modules'
- CELL, Bd.48, 1987 Seiten 271 - 279 V.L. MILLER ET AL. 'Cholera toxin transcriptional activator ToxR is a transmembrane DNA binding protein'

## Beschreibung

Die Erfindung betrifft ein isoliertes Replikon.

Bisherige Versuche, Antikörper ohne Immunisierung zu gewinnen, gründen sich auf der Präsentation von F_{ab}-Fragmenten auf Hüllproteinen des filamentösen Phagen M13 oder f1 und der Anreicherung gut bindender Varianten aus komplexen Populationen heraus durch Adsorption an immobilisiertes Hapten, gefolgt von Desorption und biologischer Vermehrung der selektiv an der Hapten-Matrix zurückgehaltenen Phagen. Diese biochemisch/genetische Anreicherungsmethode wurde hauptsächlich von R. Lerner und G. Winter populär gemacht.

Darüber hinaus ist aus Parsott und Mekalanos, J. Bakt., 173, 2842 (1991) bekannt, daß das Produkt des toxR-Gens von Vibrio cholerae (Mᵣ = 32527) verantwortlich für die koordinierte Expression mehrerer Virulenzdeterminaten, vor allem des Cholera-Toxins selbst (codiert im ctxAB-Operon), und einer Reihe andere Proteine ist. Nach einem von Miller et al. beschriebenen Modell (Cell, 48, 271 (1987)) ist ToxR ein Transmembranprotein mit periplasmatisch lokalisierter carboxyterminaler Domäne (AS202/294), einer Transmembranhelix und einer cytoplasmatisch lokalisierten aminoterminalen Domäne (AS1-182), wobei die genannte aminoterminale Domäne Homologie zu anderen bakteriellen Transkriptionsaktivatoren wie OmpR, PhoM oder PhoG zeigt. ToxR bindet dabei als Membran-verankertes Protein direkt an der Promotor-Operator-Region des ctx Operons und wirkt als Transkriptionsaktivator. Essentiell für die Bindung ist die achtfach wiederholte Sequenz TTTGAT im Operator, die im Bereich zwischen -50 und -112 liegt. Von Miller et al. konnte gezeigt werden, daß eine ToxR-vermittelte Signaltransduktion in E. coli unter Verwendung eines E. coli Stammes mit chromosomal integrierter ctxlacZ Genfusion, bei dem das Gen für β-Galactosidase (lacZ) unter Kontrolle des ctx-Promotors steht, nachgestellt werden kann. In dieser Konstruktion wurde das lacZ-Gen im durchgehenden Leseraster hinter die ersten 28 Codons des ctxA-Gens gesetzt. Wenn das Fusionsgen aus ToxR und phoA aus einem Plasmid in die Zellen gebracht wurde, konnte eine Transkriptionsaktivierung des ctx-Promotors über die β-Galactosidaseexpression nachgewiesen werden.

Nach dem von Miller beschriebenen Modell stellt die Dimerisierung der periplasmatischen ToxR-Domänen das notwendige und hinreichende Aktivierungssignal dar. Dieses Modell wurde auf dem Befund, daß ein ToxR-Derivat, bei dem die periplasmatische Domäne entfernt und durch alkalische Phosphatase, ein periplasmatisch lokalisiertes dimeres Protein, ersetzt wurde, eine Signaltransduktion vermittelte (Dauer-"on"-Zustand), begründet.

Die Aufgabe der vorliegenden Erfindung bestand nun in der Bereitstellung eines Replikons, daß ein Transkriptionssignal mit einer Immunoglobulindomäne ableiten kann.

Ein zur genetischen Selektion durch Transduktion geeigneter Mikroorganismus, der eine genetisch stabile detektierbare und/oder selektierbare Funktion enthält, wird mit einem Replikon, insbesondere einem Plasmid, Phagengenom oder Phasmid, codierend für ein Fusionsprotein aus Immunoglobulindomäne des Bence-Jones Proteins REI, ToxR Transmembranhelix und ToxR Regulationsdomäne transformiert und die durch Signaltransduktion erzeugte Funktion bestimmt und/oder auf sie selektiert.

Die Erfindung betrifft ein Replikon umfassend die Fusion von ToxR Transmembranhelix, ToxR Regulationsdomäne und die Immunglobulindomäne des Bence-Jones Proteins REI.

Bevorzugt ist weiterhin, daß die Transmembranhelix ausgewählt ist aus den Transbranhelices des ToxR Gens von Vibrio cholerae, des Protoonkogens C-neu, des P-neu Onkogens und des membranständigen IgM. Der bevorzugte Transkriptionsaktivator des Verfahrens ist das N-terminale Ende, d.h. Aminosäuren 1-182, des ToxR Proteins.

Besonders bevorzugt sind die Escherichia coli Stämme FHK11 und FHK12.

Die Erfindung wird durch die folgenden Figuren und Tabellen näher erläutert.

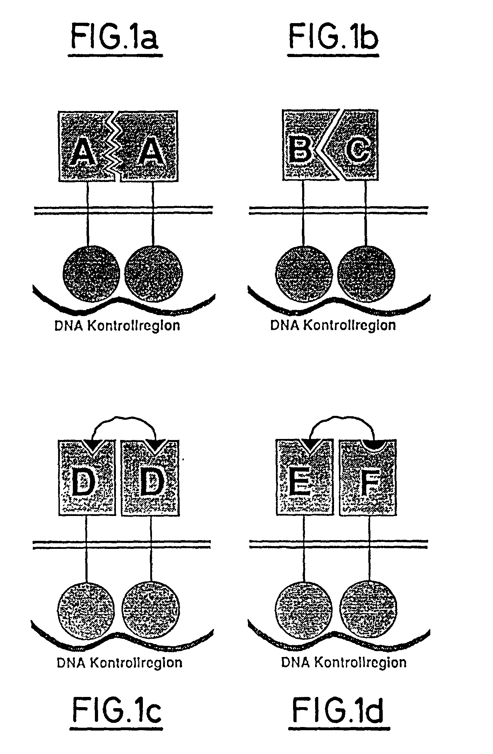

Fig. 1 Schema der Signaltransduktion durch Dimerenbildung;
a) nicht ligandeninduzierte Homodimerbildung,
b) nicht ligandeninduzierte Heterodimerbildung,
c) ligandeninduzierte Homodimerbildung und
d) ligandeninduzierte Heterodimerbildung

Fig. 2 Schematische Darstellung der Klonierungsschritte zum Aufbau des Vektors pHKToxREI.

Fig. 3 Physikalische und genetische Karte von pHKToxREI.
cat: Gen für Chloramphenicolacetyltransferase; colE1-ori: ColE1-Replikationsursprung; f1-ori: Replikationsursprung des Bakteriophagen f1; fdT: Transkriptionsterminatoren des Bakteriophagen fd; toxR: promotorproximaler Abschnitt des ToxR-Genes (Promotor einschließlich Codons 1-210); rei: Gen für die Immunglobulindomäne REI, phoA: codierende Sequenz für alkalische Phosphatase.

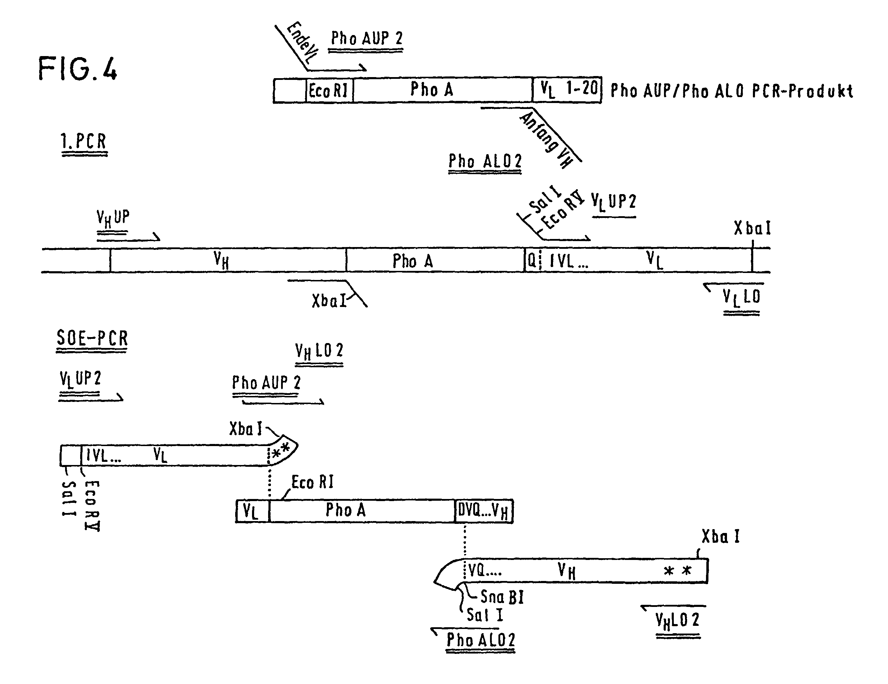

Fig. 4 PCR-Schema zur Herstellung des Konstrukts V_{L}phoAV_{H}.
Tab. 1 Sequenz des Einketten Fv-Fragmentes (scFv) des Phenyloxazolonbindenen Antikörpers NQ10.12.5.

Tab. 2 Ausschnitt aus der Nucleotidsequenz des Konstrukts POxRV_{H}PeIBV_{L}; SD: Shine-Dalgarno Sequenz; +++++ durch Pe1B1-Oligonucleotid eingeführte Sequenz; ***** durch PeIB2-Oligonucleotid eingeführte Sequenz.

Tab. 3 Sequenz des V_{H}phoAV_{L}-Konstrukts.

Tab. 4 Sequenz des V_{L}phoAV_{H}-Konstrukts.

Tab. 5 Nucleotidsequenz des Promotor-proximalen Abschnitts des toxR-Gens; die putative Transmembranhelix ist doppelt unterstrichen.

Tab. 6 Verwendete Oligonucleotide.

Die vorliegende Erfindung offenbart ein experimentelles System, das wesentliche Merkmale des Immunsystems höherer Vertebraten in Mikrooganismen nachstellt. Dieses System kann dabei zur durch Antigenbindung induzierten, klonalen Expansion und die Feinanpassung der Antigenerkennung durch Mutation und Selektion verwendet werden.

Die Erfindung wird durch die folgenden Beispiele näher erläutert.

### Beispiele

### Beispiel I (Stammkonstruktion)

### 1. Konstruktion von FHK11

### FHK11: F⁻, ara, Δ(lac-proAB), rpsL, φ80 dΔ(lacZM15), attHB::ctxDsiglacZ

Der ctx-Promotor wurde aus dem Chromosom eines pathogenen V. cholerae Stammes über PCR mittels der Oligonucleotide CtxUp und ctxLo amplifiziert. Das PCR-Produkt enthält die ctx-Promotorregion mit siebenfach wiederholter ToxR Erkennungssequenz (Miller et al., Cell 48, 271 (1987)). Da die hierin beschriebenen Escherichia coli Stämme mit chromosomal integrierter ctxlacZ-Genfusion eine genetische Instabilität zeigten, wurde abweichend von Miller et al. die putative CtxA-Signalsequenz von Codon 5 bis 28 entfernt. Dies geschah durch Reamplifikation des ctx-Promotors mit Hilfe der Oligonucleotide CTxUp und CtxΔsig.

Die zum *ctx*-Promotor komplementären Sequenzen sind durch Fettdruck hervorgehoben.

Das lacZ-Gen wurde anschließend an den ctx-Promotor via SOE-PCR fusioniert. Das resultierende Produkt wurde als BamHI Fragment in den BamHI linearisierten Vektor pLDR10 (Diederich et al., Plasmid, 28, 14-24 (1992)) in einer Orientierung, in der der ctx-Promotor gegenläufig zum Promotor des bla-Gens angeordnet ist, kloniert und nach der von Diederich beschriebenen Methode in das Chromosom des E. coli Stammes JM83 integriert.

### 2. Konstruktion von FHK12

### FHK12: F'lacZΔM15, lacY⁺, ProA⁺B⁺ ara, Δ(lac-proAB), rpsl, φ80 dΔ (lacZM15), attB::ctxDsiglacZ

In den Stamm FHK11 wurde durch Konjugation mit dem Stamm CSH22 (trpR, Δlac-pro, thi, F'lacZΔM15, lacY⁺, ProA⁺B⁺) das F-Episom dieses Stammes herübergebracht. Das F-Episom enthält das Gen für Lac-Permease (lacY) und komplementiert die chromosomale pro-Deletion. Konjuganten konnten daher leicht durch Selektion auf M9-Platten, die Ampicillin enthielten, erhalten werden. FHK12 wuchs dabei bereits ohne Aktivierung des ctx-Promotors in M9-Lactosemedium, sowie auf M9-Lactose Minimalplatten.

### Beispiel II (Replikakonstruktion)

### 1. Konstruktion von pHKToxREI

Mit Hilfe der PCR-Primer IMG212 und IMG142 wurde aus dem Zellysat eines pathogenen V. cholerae Stammes der promotorproximale Anteil des toxR-Gens (siehe Tab. 5), welches den toxR-Promotor und den Sequenzabschnitt für die ersten 210 Aminosäuren (V.L. Miller et al.: Cholera toxin transcriptional activator ToxR is a transmembrane DNA binding protein; Cell 48, 271-279 (1987)) enthält, amplifiziert. Das Reaktionsprodukt wurde mit MluI und PstI geschnitten und in ein mit MluI/PstI geschnittenes pBluescript-Derivat (pBluescriptII-pms1'; Quelle B. Fartmann, Inst. für Molekulare Genetik der Universität Göttingen) gesetzt, welches unique Restriktionsschnittstellen für BamHI, MluI und PstI (in der angegebenen Reihenfolge) aufweist. Aus diesem Konstrukt wurde das 2 kbp BamHI/PstI Fragment isoliert, welches den toxR-Gensequenzabschnitt enthält. Parallel wurde aus dem Vektor pHKREI (H. Kolmar et al., J. Mol. Biol. 228, 359-365, (1992)) ein SalI/XbaI Fragment entnommen, welches ein rei-phoA Fusionsgen (d.h. das Gen für die Immunglobulindomäne REI, sowie das Gen für Alkalische Phosphatase) enthält und in den SalI/XbaI geschnittenen Vektor pMCΔbla (H. Kolmar: Über die Faltungsstabilität einer varianten Immunglobulindomäne. Dissertation an der Eberhard-Karls-Universität Tübingen (1992)) gesetzt. Das resultierende Konstrukt (pMcΔbla-reiphoA) wurde mit BamHI und XbaI geschnitten und das resultierende Vektorfragment mit dem oben bezeichneten BamHI/XbaI Fragment, welches den ToxR-Gensequenzabschnitt enthält, ligiert. Der mitgenommene pms1'-Sequenzabschnitt wurde durch Schneiden des resultierenden Vektors, Auffüllen der Enden und Religation entfernt. In die unique HindIII-Schnittstelle des resultierenden Vektors, welche hinter dem phoA-Gen liegt, wurde ein HindIII-Fragment aus pMcΔbla-lacbla-REI (H. Kolmar: loc. cit.) eingesetzt, welches das Gen für die Immunglobulindomäne REI enthält. Durch gezielte Mutagenese mit Hilfe des Oligonucleotids IMG166 wurde anschließend die codierende Sequenz des rei-Gens (Kolmar et al., 1992) an die codierende Sequenz des toxR-Gens (Codon 1 bis 210) fusioniert und die intergenische EcoRV-Schnittstelle eingeführt (pHKToxREI). Relevante Resbiktionsschninstellen sind unterstrichen, die zur *toxR*-Region aus *V. cholerae* komplementären Sequenzabschnitte sind durch Fettdruck hervorgehoben.

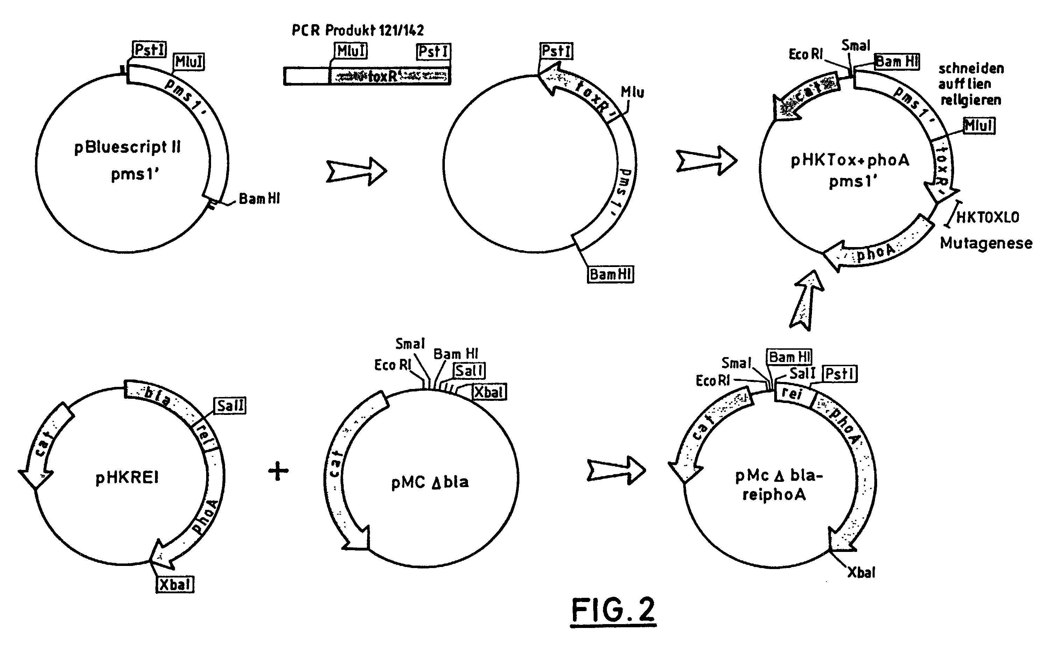

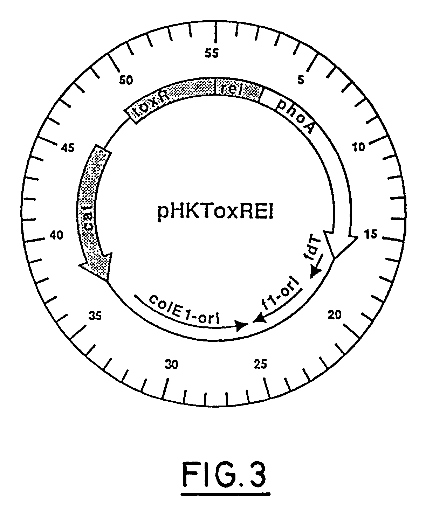

Eine schematische Darstellung der vorstehenden Klonierungsschritte zum Aufbau des Vektors pHKToxREI ist in Figur 2, eine physikalische und genetische Karte von pHK-Tox-REI in Figur 3 dargestellt.

### 2. Konstruktion von pHKToxscFv

Das Einketten-Fv-Fragment (scFv) des Phenyloxazolon-bindenden Antikörpers NQ10.12.5 (Berek et al., 1985; Berek und Milstein, 1987; Sequenz: siehe Tab. 1), bei dem der C-Terminus der V_{H}-Domäne über einen kurzen Peptid-Linker [(Gly₄Ser)₃] mit dem N-Terminus der V_{L}-Domäne kovalent verbunden ist, wurde von der Arbeitsgruppe Greg Winter (MRC, Cambridge) in Form des Plasmids pHEN1::NQ10.12.5scFv-Fragment als SfiI/NotI-Fragment in den Vektor pHEN1 (H.R. Hoogenboom et al.: Multisubunit proteins on the surface of filamentous phage: methodologies for displaying antibody (Fab) heavy and light chains; Nucl. Acids Res. 19, 4133-4137 (1991)) kloniert zur Verfügung gestellt. Es wurde zunächst einschließlich der davor befindlichen pelB-Leadersequenz als HindIII/NotI-Fragment in den Bluescript-Vektor pBSK(-) umkloniert und einer Nucleotid-Sequenzanalyse unterzogen. Das klonierte scFv-Gen wich in einigen Bereichen stark von der publizierten NQ10.12.5-Sequenz (Berek et al, 1985) ab. Dies betraf vor allem den Anfang und das Ende der codierenden Bereiche für V_{H} und V_{L}. Die Abweichungen in diesen Bereichen sind möglicherweise auf die Verwendung degenerierter Primer bei der Amplifikation dieser Gene aus der NQ10.12.5 Zellinie zurückzuführen. Darüber hinaus wurden in der codierenden Sequenz für V_{H} und V_{L} (neben einigen stillen Basenaustauschen) noch jeweils zwei Punktmutationen sequenziert, die zu Aminosäureaustauschen führten (V_{H}: I57->L, T77->N; V_{L}:K18->R, T48->I).

Die Reparatur des scFv-Gens erfolgte nach folgender Strategie:
* Die codierenden Bereiche für V_{H} und V_{L} wurden zunächst getrennt mittels PCR reamplifiziert. Durch die PCR-Primer wurden die Sequenzen am Beginn und Ende dieser Bereiche zugunsten der NQ10.12.5-Sequenz repariert. Durch den V_{H}UP-Primer wurde vor dem V_{H}-Gen eine SalI-Schnittstelle unddurch den V_{H}LO-Primer im Bereich der codierenden Sequenz für den single chain-Linker eine BamHI-Schnittstelle eingeführt. Diese BamHI-Schnittstelle wurde ebenfalls durch den V_{L}UP-Primer eingeführt. Schließlich wurde am Ende des V_{L}-Gens durch den V_{L}LO-Primer eine XbaI-Schnittstelle eingeführt.
* Mit Hilfe dieser Schnittstellen wurden die amplifizierten Fragmente zunächst getrennt in den pBSK(-)-Vektor kloniert (die am Beginn des V_{H}-Gens eingeführte SnaBI (blunt)-Schnittstelle wurde für die spätere Klonierung des scFV-Gens hinter das toxR-Gen benötigt, das am Ende eine EcoRV-Schnittstelle (ebenfalls blunt) besitzt).
* An den getrennt klonierten Fragmenten erfolgte zunächst die Reparatur der 4 verbliebenen Punktmutationen durch Oligonucleotid-dirigierte Mutagenese. Aufgrund der Entfernung der einzelnen Mutationen zueinander mußte für jede Mutagenese ein sparates Reparatur-Oligonucleotid definiert werden. Um das screening zu erleichtern wurden die Oligonucleotide so definiert, daß sie zusätzlich zur Reparaturmutagenese eine Restriktions-Schnittstelle einführten bzw. zerstörten.
* Schließlich wurden die Fragmente mit Hilfe der in der codierenden Sequenz für den single chain-Linker liegenden BamHI-Schnittstelle wieder zusammen kloniert.

Das NQ10.12.5 scFv-Gen wurde als SnaBi/XbaI-Fragment (Sequenz siehe Tab. 1) in den mit EcoRV und XbaI geschnittenen Vaktor pHKToxREI kloniert. Mit EcoRV und XbaI wird die für REI und PhoA codierende Sequenz aus diesem Plasmid herausgespalten. Die Klonierung des scFv-Gens in den mit EcoRV und XbaI geschnittenen Vektor pHKToxRei resultiert in einem toxR-scFv-Fusionsgen.

### 3. Konstruktion von pHKToxV_{H}phoAV_{L}

Des weiteren wurde eine Fusion aus ToxR und dem Zweiketten-Fv-Fragment des Antikörpers NQ10.12.5 konstruiert, bei dem die V_{H}-Domäne am Carboxyterminus des ToxR-Proteins belassen wurde, während V_{L} in löslicher Form coexprimiert wird. Um die Sekretion des V_{L}-Proteins ins Periplasma zu ermöglichen, solte das V_{L}Gen mit der pelB-Leader-Sequenz (S.-P. Lei et al., (1987): Characterization of the Erwinia carotovora pelB Gene and its product pectat lyase; J. Bacteriol. 169, 4379-4383) versehen werden. Die Entfernung des single chain-Linkers und die Einführung des Leaders sollte durch zwei aufeinanderfolgende Oligonucleotid-dirigierte Mutagenesen mit Hilfe der Oligonucleotide PelB1 und PelB2 erfolgen.

Nach der zweiten Mutagenese wurde ein Klon erhalten, der das korrekte Restriktionsmuster aufwies. Die Nucleotid-Sequenzanalyse dieses Klons ergab jedoch drei Deletionen, zwei (1 bzw. 8 Nucleotide) in der codierenden Sequenz für das Signalpeptid und eine (1 Nucleotid) in der intergenischen Region zwischsen V_{H} und pelBV_{L} (siehe Tab. 2).

Die fehlerhafte pelB-Leadersequenz vor dem V_{L}-Gen wurde daraufhin durch die Leadersequenz der Alkalischen Phosphatase (H. Inouye et al.: Signal sequence of alkaline phosphatase of Escherichia coli; J. Bacteriol. 149, 434-439, (1982)) ersetzt. Zur Amplifikation der phoA-Signalsequenz aus dem E. coli-Chromosom wurden die PCR-Primer PhoASigUP und PhoASigLO verwendet.

Das 5'-Ende des PhoASigLO-Primers war homolog zum promotorproximalen Bereich des V_{L}-Gens, so daß das phoA-Fragment mittels SOE-OPCR mit dem V_{L}-Gen verknüpft werden konnte. Am 5'-Ende des PhoASigUP-Primers wurde eine EcoRI-Schnittstelle definiert, mit Hilfe derer das phoA-VL SOE-PCR Produkt hinter das V_{H}-Gen kloniert wurde. Die Sequenz des V_{H}phoAV_{L}-Konstrukts ist in Tab. 3 wiedergegeben.

### 4. Konstruktion von pHKToxV_{L}phoAV_{H}

Die Konstruktion von pHKToxV_{L}phoAV_{H}, bei dem das V_{L}-Gen mit der toxR-Sequenz fusioniert und V_{H} mit der phoA-Leadersequenz versehen wurde, erfolgte mittels PCR mit Hilfe der Oligonucleotide IMG409 - IMG 412 (siehe Fig. 4).

Der codierende Bereich für die phoA-Signalsequenz (H. Inouye et al.: Signal sequence of alkaline phosphatase of Escherichia coli; J. Bacteriol. 149, 434-439 (1982)) wurde zunächst mit Hilfe der Primer PhoAUP2 und PhoALO2 amplifiziert. Als Template diente hierbei das mit Hilfe der Primer PhoASigUP und PhoASigLO aus dem Chromosom von E. coli ammplifizierte PCR-Produkt (siehe Beispiel II.3 "Konstruktion von pHKToxV_{H}phoAV_{L}"). Der 5'-Terminus des Primer PhoAUP2 war komplementär zum Ende der V_{L}-Sequenz, der 5'-Terminus des Primer PhoAL02 zu den ersten 20 Nucleotiden der V_{H}-Sequenz. Die komplementären Enden wurden im nächsten Schritt für die SOE-PCR mit dem V_{H}- bzw. V_{L}-Fragment benötigt. Weiterhin wurde durch den PhoALO2-Primer der N-terminale Aspartatrest der NQ10.12.5-V_{H}-Sequenz (P.M. Alzari et al.: Three-dimensional structure determination of an anti-2-phenyloxazolone antibody: the role of somatic mutation and heavy/light chain pairing in the maturation of an immune response; EMBO J., 9, 3807-3814 (1990)), der in den bisherigen Konstrukten aus praktischen Überlegungen (Notwendigkeit einer Restriktionsschnittstelle) fehlte, wieder eingeführt.

Das V_{H}-Gen wurde mit Hilfe der Primer V_{H}UP und V_{H}LO2, das V_{L}-Gen mit den Primern V_{L}UP2 und V_{L}LO aus dem V_{H}PhoAV_{L}-Konstrukt amplifiziert. Die Primer V_{L}UP2 und V_{H}LO2 führten die für die Klonierungen notwendigen Schnittstellen ein (SalI und XbaI für die Klonierung in den Bluescript-Vektor pBSK(-), EcoRV und XbaI für die Klonierung in pHKToxREI). Da am Beginn der V_{L}-Sequenz eine blunt-Schnittstelle benötigt wurde, konnte das erste Codon (Gln) nicht mit amplifiziert werden. Durch Austausch des ATT-Codons # 2 gegen ATC (codieren beide für Ile) war es möglich, eine EcoRV-Schnittstelle zu erzeugen. Die drei PCR-PRodukte wurden anschließend durch SOE-PCR miteinander verknüpft. Die Sequenz des V_{L}phoAV_{H}-Konstrukts ist in Tab. 4 wiedergegeben. Das SOE-PCR Produkt wurde anschließend als EcoRV/XbaI-Fragment in den Vektor pHKToxREI kloniert. Hierbei wurde die für REI und PhoA codierende Sequenz entfernt und V_{L} in frame mit der für ToxR codierenden Sequenz fusioniert.

### Beispiel III

Signaltransduktion eines homodimeren Fusionsproteins bestehend aus der Transkriptionsaktivatordomäne von ToxR und einer Immunglobulin variablen leichten Kette.

Es wurde der Vektor pHKToxREI wie in Beispiel II Punkt 1 beschrieben aufgebaut. Dieser enthält ein Fusionsgen aus der Transkriptionsaktivatordomäne von ToxR und dem Gen für die variable Immunglobulindomäne des Bence-Jones Proteins REI (H. Kolmar et al.: J. Mol. Biol. 228, 359-365 (1992)). Die REI-Domäne ist ein Homodimer (Epp et al., Eur. J. Biochem. 45, 513-524, (1974)). Als Kontrolle wurde der Vektor pHKTox-TAG konstruiert, bei dem durch gezielte Mutagenese mit Hilfe des Oligonucleotids IMG167 zwischen toxR und rei ein Stopcodon inseriert wurde, so daß ausgehend von diesem Vektor lediglich die Transkriptionsaktivatordomäne, nicht jedoch die REI Domäne exprimiert wird.

Nach Transformation des Stammes FHK11 mit diesem Vektor wurde die Aktivierung des durch Dimerisierung der extracytoplasmatischsen REI-Domänen vermittelten chromosomal integrierten ctx-Promotors durch Messung der β-Galactosidaseaktivität in den bei 37 °C angezogenen Übernachtkulturen der betreffenden Transformanten bestimmt. Die Enzymaktivität betrug für pHKToxTAG (fehlende extracytoplasmatische Domäne) 130 Miller Einheiten, für pHKToxREI hingegen 400 Miller Einheiten, was einer ca. dreifachen Transkripitonsaktivierung entspricht. Hiermit ist gezeigt, daß die Dimerisierung der extracytoplasmatischen Immunglobulindomänen über die ToxR-vermittelte Signaltransduktion direkt detektiert werden kann.

Der E. coli Stamm FHK12/pHKToxV_{L}phoAV_{H} wurde am 07. Juni 1993 bei der Deutschen Sammlung von Mikroorganismen und Zellkulturen GmbH, Maschroder Weg 1b, W3300 Braunschweig unter der Bezeichnung DSM 8345 hinterlegt.

### SEQUENZPROTOKOLL

(1) ALGEMEINE INFORMATION:
   (i) ANMELDER:
      (A) NAME: Behringwerke AG
      (B) STRASSE: Postfach 1140
      (C) ORT: Marburg
      (E) LAND: Deutschland
      (F) POSTLEITZAHL: 35001
   (ii) ANMELDETITEL: Genetische Selektion von zur Ligandenbindung befaehigten Proteinen mittels Signaltransduktion in Mikroorganismen
   (iii) ANZAHL DER SEQUENZEN: 33
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 738 Basenpaare
      (B) ART: Nukleinsäure
      (C) STRANGFORM: Doppel
      (D) TOPOLOGIE: nicht bekannt
   (ii) ART DES MOLEKÜLS: DNS (genomisch)
   (ix) MERKMALE:
      (A) NAME/SCHLÜSSEL: CDS
      (B) LAGE: 1..720
   (xi) SEQUENZBESCHREIBUNG: SEQ ID NO: 1:
   CAG CGA GTC GAC TAC GTA CAG CTG GTG GAG CTT GGG GGA GGC TTT GTG        48
   Gln Arg Val Asp Tyr Val Gln Leu Val Glu Leu Gly Gly Gly Phe Val
     1               5                  10                  15
   CAG CCT GGA GGG TCC CGG AAA CTC TCC TGT GCA GCC TCT GGA TTC ACT        96
   Gln Pro Gly Gly Ser Arg Lys Leu Ser Cys Ala Ala Ser Gly Phe Thr
                20                  25                  30
   TTC AGT AGC TTT GGA ATG CAC TGG GTT CGT CAG GCT CCA GAG AAG GGG       144
   Phe Ser Ser Phe Gly Met His Trp Val Arg Gln Ala Pro Glu Lys Gly
            35                  40                  45
   CTG GAG TGG GTC GCA TAT ATT AGT AGT GGC AGT AGT ACT ATC TAC TAT       192
   Leu Glu Trp Val Ala Tyr Ile Ser Ser Gly Ser Ser Thr Ile Tyr Tyr
        50                  55                  60
   GCA GAC ACA GTG AAG GGC CGA TTC ACC ATC TCC AGA GAC AAT CCC AAG       240
   Ala Asp Thr Val Lys Gly Arg Phe Thr Ile Ser Arg Asp Asn Pro Lys
    65                  70                  75                      80
   AAC ACC CTG TTC CTG CAG ATG ACC AGT CTA AGG TCT GAG GAC ACG GCC       288
   Asn Thr Leu Phe Leu Gln Met Thr Ser Leu Arg Ser Glu Asp Thr Ala
                    85                  90                  95
   ATG TAT TAC TGT GCA AGA GAT TAC GGG GCT TAT TGG GGC CAA GGG ACC       336
   Met Tyr Tyr Cys Ala Arg Asp Tyr Gly Ala Tyr Trp Gly Gln Gly Thr
               100                 105                 110
   CTG GTC ACC GTC TCC TCA GGT GGA GGC GGT TCA GGC GGA GGT GGC TCT       384
   Leu Val Thr Val Ser Ser Gly Gly Gly Gly Ser Gly Gly Gly Gly Ser
           115                 120                 125
   GGC GGT GGC GGA TCC CAA ATT GTT CTC ACC CAG TCT CCA GCA ATC ATG       432
   Gly Gly Gly Gly Ser Gln Ile Val Leu Thr Gln Ser Pro Ala Ile Met
       130                 135                 140
   TCT GCA TCT CCA GGG GAG AAA GTC ACC ATG ACC TGC AGT GCC AGT TCA       480
   Ser Ala Ser Pro Gly Glu Lys Val Thr Met Thr Cys Ser Ala Ser Ser
   145                 150                 155                 160
   AGT GTA AGG TAC ATG AAC TGG TTC CAA CAG AAG TCA GGC ACC TCC CCC       528
   Ser Val Arg Tyr Met Asn Trp Phe Gln Gln Lys Ser Gly Thr Ser Pro
                   165                 170                 175
   AAG CGC TGG ACA TAT GAC ACA TCC AAA CTG TCT TCT GGA GTC CCT GCT       576
   Lys Arg Trp Thr Tyr Asp Thr Ser Lys Leu Ser Ser Gly Val Pro Ala
               180                 185                 190
   CGC TTC AGT GGC AGT GGG TCT GGG ACC TCT TAC TCT CTC ACA ATC AGC       624
   Arg Phe Ser Gly Ser Gly Ser Gly Thr Ser Tyr Ser Leu Thr Ile Ser
           195                 200                 205
   AGC ATG GAG GCT GAA GAT GCT GCC ACT TAT TAC TGC CAG CAG TGG AGT       672
   Ser Met Glu Ala Glu Asp Ala Ala Thr Tyr Tyr Cys Gln Gln Trp Ser
       210                 215                 220
   AGT AAT CCA CTC ACT TTC GGT GCT GGG ACC AAG CTT GAA CTG AAA CGG       720
   Ser Asn Pro Leu Thr Phe Gly Ala Gly Thr Lys Leu Glu Leu Lys Arg
   225                 230                 235                 240
   TGATAATCTA GAACCGCG                                                   738
(2) INFORMATION ZU SEQ ID NO: 2:
   (i) SEQUENZ CHARAKTERISTIKA:
      (A) LÄNGE: 240 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Gln Arg Val Asp Tyr Val Gln Leu Val Glu Leu Gly Gly Gly Phe Val
    1               5                  10                  15
   Gln Pro Gly Gly Ser Arg Lys Leu Ser Cys Ala Ala Ser Gly Phe Thr
               20                  25                  30
   Phe Ser Ser Phe Gly Met His Trp Val Arg Gln Ala Pro Glu Lys Gly
           35                  40                  45
   Leu Glu Trp Val Ala Tyr Ile Ser Ser Gly Ser Ser Thr Ile Tyr Tyr
       50                  55                  60
   Ala Asp Thr Val Lys Gly Arg Phe Thr Ile Ser Arg Asp Asn Pro Lys
   65                  70                  75                  80
   Asn Thr Leu Phe Leu Gln Met Thr Ser Leu Arg Ser Glu Asp Thr Ala
                   85                  90                  95
   Met Tyr Tyr Cys Ala Arg Asp Tyr Gly Ala Tyr Trp Gly Gln Gly Thr
               100                 105                 110
   Leu Val Thr Val Ser Ser Gly Gly Gly Gly Ser Gly Gly Gly Gly Ser
           115                 120                 125
   Gly Gly Gly Gly Ser Gln Ile Val Leu Thr Gln Ser Pro Ala Ile Met
       130                 135                 140
   Ser Ala Ser Pro Gly Glu Lys Val Thr Met Thr Cys Ser Ala Ser Ser
   145                 150                 155                 160
   Ser Val Arg Tyr Met Asn Trp Phe Gln Gln Lys Ser Gly Thr Ser Pro
                   165                 170                 175
   Lys Arg Trp Thr Tyr Asp Thr Ser Lys Leu Ser Ser Gly Val Pro Ala
               180                 185                 190
   Arg Phe Ser Gly Ser Gly Ser Gly Thr Ser Tyr Ser Leu Thr Ile Ser
           195                 200                 205
   Ser Met Glu Ala Glu Asp Ala Ala Thr Tyr Tyr Cys Gln Gln Trp Ser
       210                 215                 220
   Ser Asn Pro Leu Thr Phe Gly Ala Gly Thr Lys Leu Glu Leu Lys Arg
   225                 230                 235                 240
(2) INFORMATION ZU SEQ ID NO: 3:
   (i) SEQUENZ CHARAKTERISTIKA:
      (A) LÄNGE: 793 Basenpaare
      (B) ART: Nukleinsäure
      (C) STRANGFORM: Doppel
      (D) TOPOLOGIE: nicht bekannt
   (ii) ART DES MOLEKÜLS: DNS (genomisch)
(2) INFORMATION ZU SEQ ID NO: 4:
   (i) SEQUENZ CHARAKTERISTIKA:
      (A) LÄNGE: 784 Basenpaare
      (B) ART: Nukleinsäure
      (C) STRANGFORM: Doppel
      (D) TOPOLOGIE: nicht bekannt
   (ii) ART DES MOLEKÜLS: DNS (genomisch)
   (xi) SEQUENZBESCHREIBUNG: SEQ ID NO: 4:
   ACAGTCGACG ATATCGTTCT CACCCAGTCT CCAGCAATCA TGTCTGCATC TCCAGGGGAG    60
   AAAGTCACCA TGACCTGCAG TGCCAGTTCA AGTGTAAGGT ACATGAACTG GTTCCAACAG   120
   AAGTCAGGCA CCTCCCCCAA GCGCTGGACA TATGACACAT CCAAACTGTC TTCTGGAGTC   180
   CCTGCTCGCT TCAGTGGCAG TGGGTCTGGG ACCTCTTACT CTCTCACAAT CAGCAGCATG   240
   GAGGCTGAAG ATGCTGCCAC TTATTACTGC CAGCAGTGGA GTAGTAATCC ACTCACTTTC   300
   GGTGCTGGGA CCAAGCTTGA ACTGAAACGG TGATAAGAAT TCATTTGTAC ATGGAGAAAA   360
   TAAAGTGAAA CAAAGCACTA TTGCACTGGC ACTCTTACCG TTACTGTTTA CCCCTGTGAC   420
   AAAAGCCGAT GTACAGCTGG TGGAGCTTGG GGGAGGCTTT GTGCAGCCTG GAGGGTCCCG   480
   GAAACTCTCC TGTGCAGCCT CTGGATTCAC TTTCAGTAGC TTTGGAATGC ACTGGGTTCG   540
   TCAGGCTCCA GAGAAGGGGC TGGAGTGGGT CGCATATATT AGTAGTGGCA GTAGTACTAT   600
   CTACTATGCA GACACAGTGA AGGGCCGATT CACCATCTCC AGAGACAATC CCAAGAACAC   660
   CCTGTTCCTG CAGATGACCA GTCTAAGGTC TGAGGACACG GCCATGTATT ACTGTGCAAG   720
   AGATTACGGG GCTTATTGGG GCCAAGGGAC CCTGGTCACC GTCTCCTCAT GATAATCTAG   780
   ATTC                                                                784
(2) INFORMATION ZU SEQ ID NO: 5:
   (i) SEQUENZ CHARAKTERISTIKA:
      (A) LÄNGE: 31 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 5:
   CGGGTCATAC CGATCCCGTT ATCCGAAATG G    31
(2) INFORMATION ZU SEQ ID NO: 6:
   (i) SEQUENZ CHARAKTERISTIKA:
      (A) LÄNGE: 39 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 6:
   CGACGGTACC TGCAGCGTTA GGGGTTTAAA GCTGGATTG 39
(2) INFORMATION ZU SEQ ID NO: 7:
   (i) SEQUENZ CHARAKTERISTIKA:
      (A) LÄNGE: 29 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 7:
   CATCTGGATA TCCGTTAGGG GTTTAAAGC 29
(2) INFORMATION ZU SEQ ID NO: 8:
   (i) SEQUENZ CHARAKTERISTIKA:
      (A) LÄNGE: 27 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 8:
   CATCTGGATA TCCTACCAAT GCTTAAT 27
(2) INFORMATION ZU SEQ ID NO: 9:
   (i) SEQUENZ CHARAKTERISTIKA:
      (A) LÄNGE: 83 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 9:
   AATTTGGGAT CCGCCACCGC CAGAGCCACC TCCGCCTGAA CCGCCTCCAC CTGAGGAGAC 60
   GGTGACCAGG GTCCCTTGGC CCC 83
(2) INFORMATION ZU SEQ ID NO: 10:
   (i) SEQUENZ CHARAKTERISTIKA:
      (A) LÄNGE: 37 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 10:
   GGTGGCGGAT CCCAAATTGT TCTCACCCAG TCTCCAG 37
(2) INFORMATION ZU SEQ ID NO: 11:
   (i) SEQUENZ CHARAKTERISTIKA:
      (A) LÄNGE: 66 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 11:
   CAGCGAGTCG ACTACGTACA GCTGGTGGAG CTTGGGGGAG GCTTTGTGCA GCCTGGAGGG 60
   TCCCGG 66
(2) INFORMATION ZU SEQ ID NO: 12:
   (i) SEQUENZ CHARAKTERISTIKA:
      (A) LÄNGE: 49 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 12:
   CGCGGTTCTA GATTATCACC GTTTCAGTTC AAGCTTGGTC CCAGCACCG 49
(2) INFORMATION ZU SEQ ID NO: 13:
   (i) SEQUENZ CHARAKTERISTIKA:
      (A) LÄNGE: 32 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 13:
   GTCTGCATAG TAGATAGTAC TACTGCCACT AC 32
(2) INFORMATION ZU SEQ ID NO: 14:
   (i) SEQUENZ CHARAKTERISTIKA:
      (A) LÄNGE: 34 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 14:
   GACTGGTCAT CTGCAGGAAC AGGGTGTTCT TGGG 34
(2) INFORMATION ZU SEQ ID NO: 15:
   (i) SEQUENZ CHARAKTERISTIKA:
      (A) LÄNGE: 26 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 15:
   GGTCATGGTG ACTTTCTCCC CTGGAG 26
(2) INFORMATION ZU SEQ ID NO: 16:
   (i) SEQUENZ CHARAKTERISTIKA:
      (A) LÄNGE: 34 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 16:
   GGATGTGTCA TATGTCCAGC GCTTGGGGGA GGTG 34
(2) INFORMATION ZU SEQ ID NO: 17:
   (i) SEQUENZ CHARAKTERISTIKA:
      (A) LÄNGE: 79 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 17:
   GAGAACAATT TGGGCCATGG CTGGTTGGGC AGCGAGTAAT AACAATCCAG CGGCTGCCGT    60
   GATATCTGAG GAGACGGTG                                                 79
(2) INFORMATION ZU SEQ ID NO: 18:
   (i) SEQUENZ CHARAKTERISTIKA:
      (A) LÄNGE: 80 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 18:
   CAGCGGCTGC CGTAGGCAAT AGGTATTTCA TTATGACTGT CTCCTTGAAA TAGAATTCGC    60
   ATTATCATGA GGAGACGGTG                                                80
(2) INFORMATION ZU SEQ ID NO: 19:
   (i) SEQUENZ CHARAKTERISTIKA:
      (A) LÄNGE: 31 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 19:
   CACGACGTTG TAGTACTACC TTTACCATAT A                                   31
(2) INFORMATION ZU SEQ ID NO: 20:
   (i) SEQUENZ CHARAKTERISTIKA:
      (A) LÄNGE: 45 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 20:
   TTGGCTTGGG TTGATCAGGA TCCCAAGCTA GCTCGATTCC CCAAG 45
(2) INFORMATION ZU SEQ ID NO: 21:
   (i) SEQUENZ CHARAKTERISTIKA:
      (A) LÄNGE: 50 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 21:
   TCGAGCTAGC CCGGTTACCT TCATCATCGC TACCGTTGAA GGAGTACTGC 50
(2) INFORMATION ZU SEQ ID NO: 22:
   (i) SEQUENZ CHARAKTERISTIKA:
      (A) LÄNGE: 59 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 22:
   ATCAGGATCC CAACCACGAC AACCAGGATC AGGAACAGCA GTACTCCAAC AACGGTAGC 59
(2) INFORMATION ZU SEQ ID NO: 23:
   (i) SEQUENZ CHARAKTERISTIKA:
      (A) LÄNGE: 42 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 23:
   GACTGGGTGA GAACAATTTG GGCTTTTGTC ACAGGGGTAA AC 42
(2) INFORMATION ZU SEQ ID NO: 24:
   (i) SEQUENZ CHARAKTERISTIKA:
      (A) LÄNGE: 38 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 24:
   TTTGAATTCA TTTGTACATG GAGAAAATAA AGTGAAAC 38
(2) INFORMATION ZU SEQ ID NO: 25:
   (i) SEQUENZ CHARAKTERISTIKA:
      (A) LÄNGE: 36 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 25:
   GAACTGAAAC GGTGATAAGA ATTCATTTGT ACATGG 36
(2) INFORMATION ZU SEQ ID NO: 26:
   (i) SEQUENZ CHARAKTERISTIKA:
      (A) LÄNGE: 32 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 26:
   ACAGTCGACG ATATCGTTCT CACCCAGTCT CC 32
(2) INFORMATION ZU SEQ ID NO: 27:
   (i) SEQUENZ CHARAKTERISTIKA:
      (A) LÄNGE: 39 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 27:
   CCAAGCTCCA CCAGCTGTAC ATCGGCTTTT GTCACAGGG 39
(2) INFORMATION ZU SEQ ID NO: 28:
   (i) SEQUENZ CHARAKTERISTIKA:
      (A) LÄNGE: 28 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 28:
   GAATCTAGAT TATCATGAGG AGACGGTG   28
(2) INFORMATION ZU SEQ ID NO: 29:
   (i) SEQUENZ CHARAKTERISTIKA:
      (A) LÄNGE: 53 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 29:
   GTGTGTGATA CGAAACGAAG CATTGGATCC TAGAAGTGAA ACGGGGTTTA CCG 53
(2) INFORMATION ZU SEQ ID NO: 30:
   (i) SEQUENZ CHARAKTERISTIKA:
      (A) LÄNGE: 55 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 30:
   GTTTTCCCAG TCACGACGAC GTTGTAAAAC GACAGAATCT GCCCGATATA TTATC 55
(2) INFORMATION ZU SEQ ID NO: 31:
   (i) SEQUENZ CHARAKTERISTIKA:
      (A) LÄNGE: 31 Basenpaare
      (B) ART: Nukleinsäure
      (C) STRANGFORM: nicht bekannt
      (D) TOPOLOGIE: nicht bekannt
   (ii) ART DES MOLEKÜLS: DNS (genomisch)
   (xi) SEQUENZBESCHREIBUNG: SEQ ID NO: 31:
   CAGCACGTTG TAGTACTACC TTTACCATAT A                                31
(2) INFORMATION ZU SEQ ID NO: 32:
   (i) SEQUENZ CHARAKTERISTIKA:
      (A) LÄNGE: 180 Basenpaare
      (B) ART: Nukleinsäure
      (C) STRANGFORM: Doppel
      (D) TOPOLOGIE: nicht bekannt
   (ii) ART DES MOLEKÜLS: DNS (genomisch)
   (xi) SEQUENZBESCHREIBUNG: SEQ ID NO: 32:
   GCAAGAGATT ACGGGGCTTA TTGGGGCCAA GGGACCCTGG TCACCGTCTC CTCATGATAA    60
   TGCGAATTCT ATTTCAAGGA GACAGTCATA ATGAAATACC TATTGCCTAC GGCAGCCGCT   120
   GGATTGTTAT TACTCGCTGC CCAACCAGCC ATGGCCCAAA TTGTTCTCAC CCAGTCTCCA   180
(2) INFORMATION ZU SEQ ID NO: 33:
   (i) SEQUENZ CHARAKTERISTIKA:
      (A) LÄNGE: 959 Basenpaare
      (B) ART: Nukleinsäure
      (C) STRANGFORM: Doppel
      (D) TOPOLOGIE: nicht bekannt
   (ii) ART DES MOLEKÜLS: DNS (genomisch)
   (xi) SEQUENZBESCHREIBUNG: SEQ ID NO: 33:

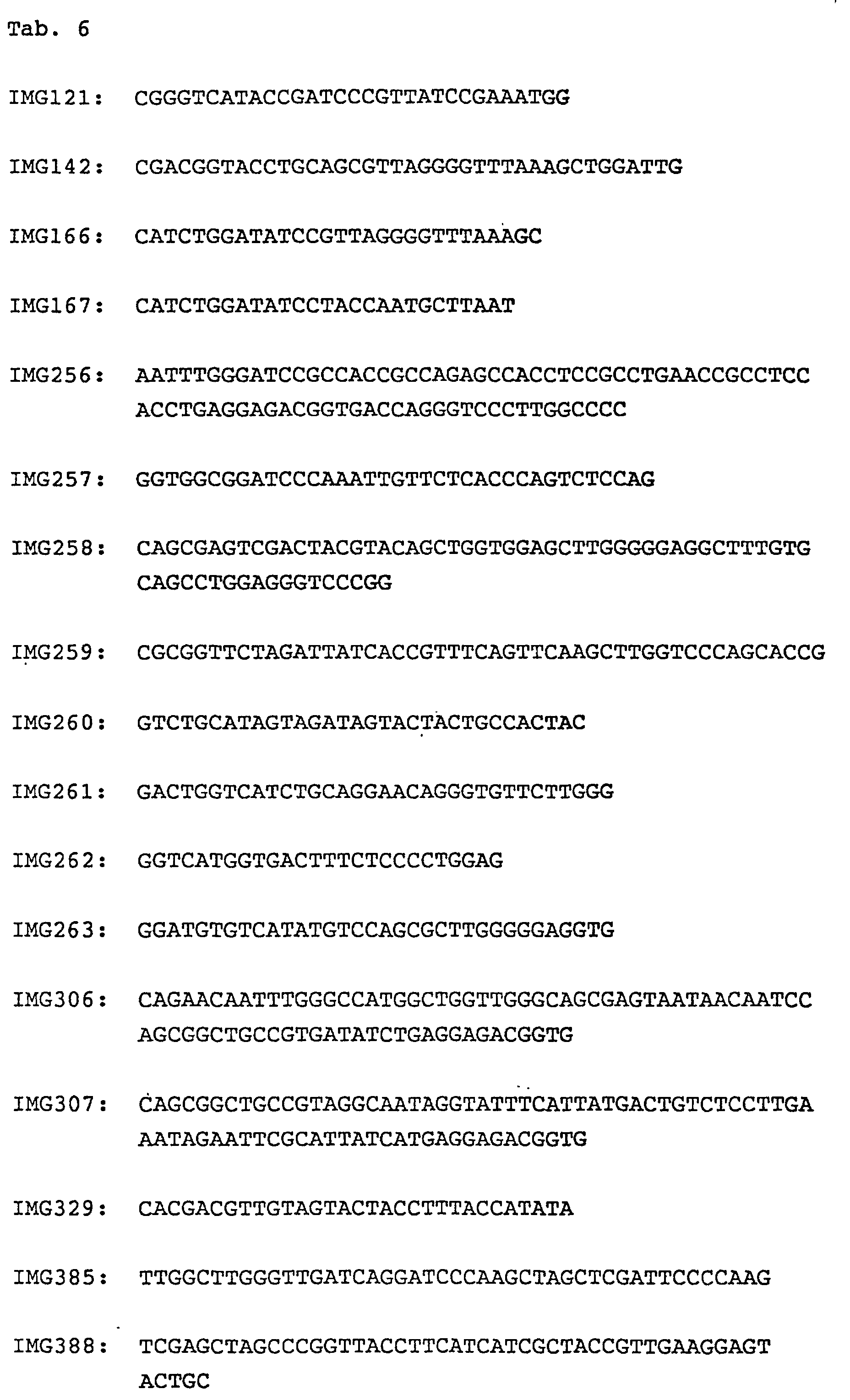

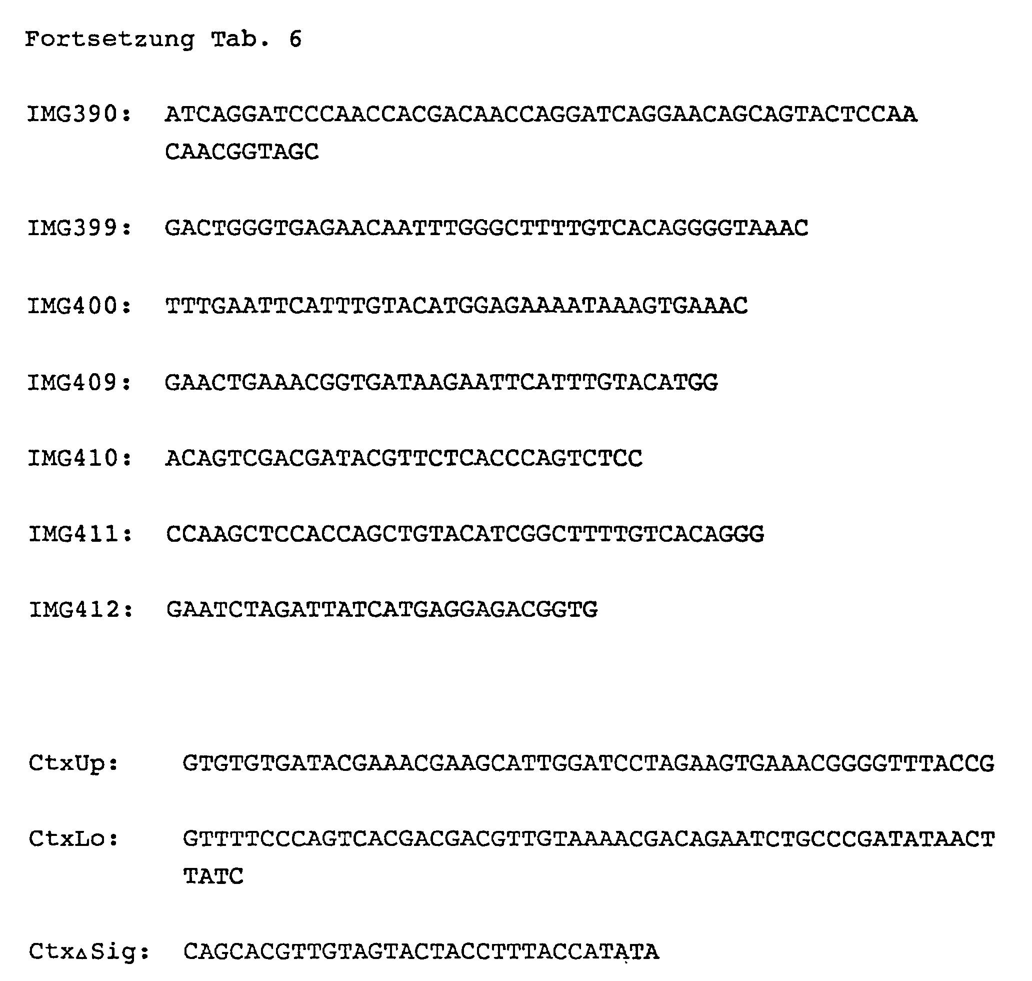

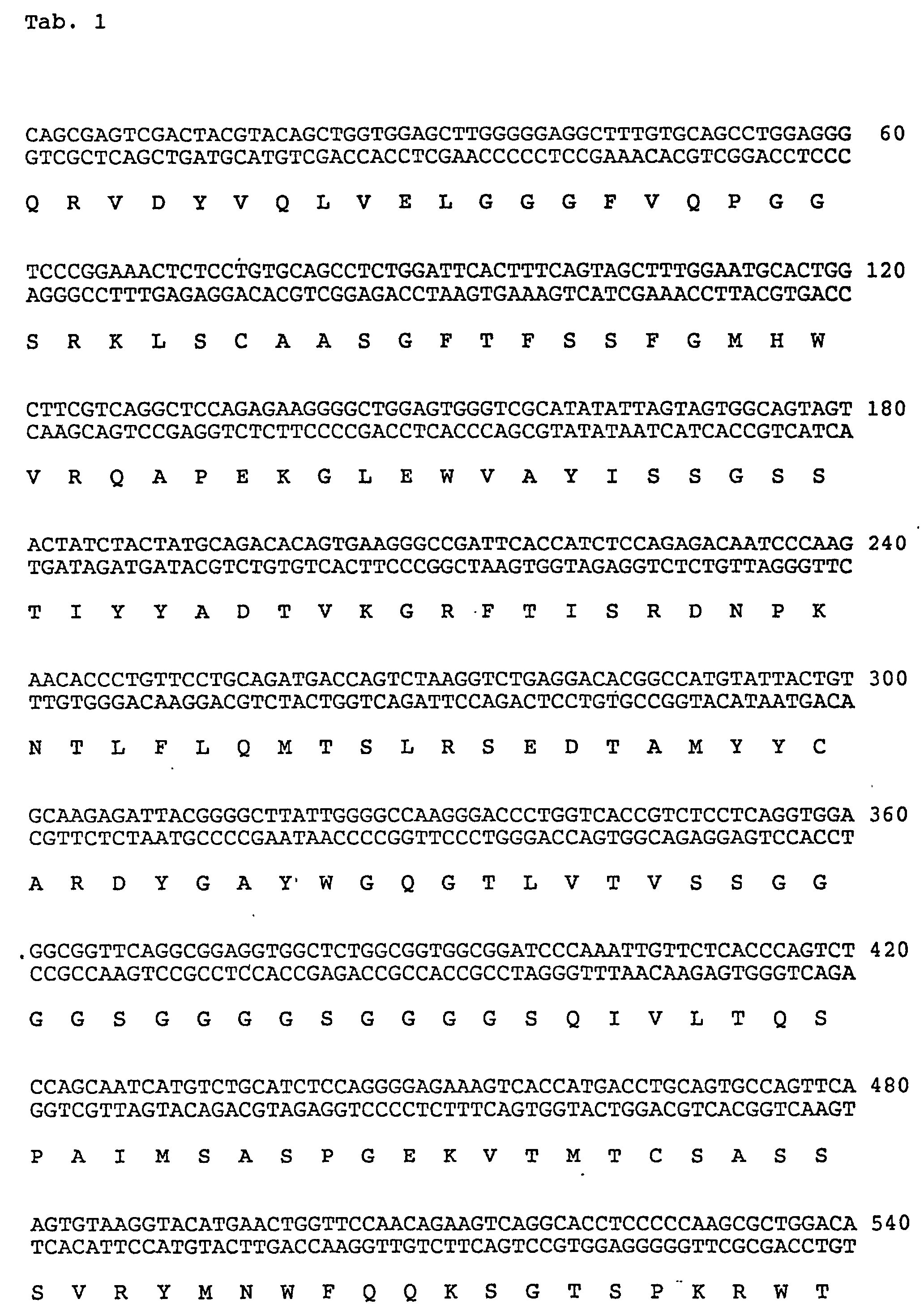

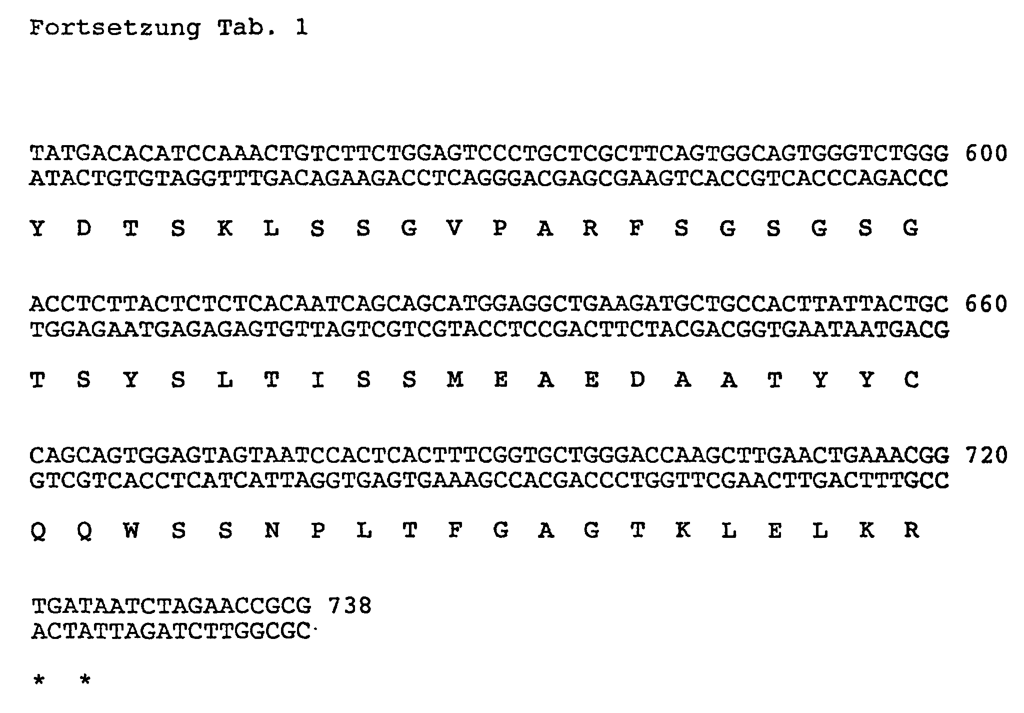

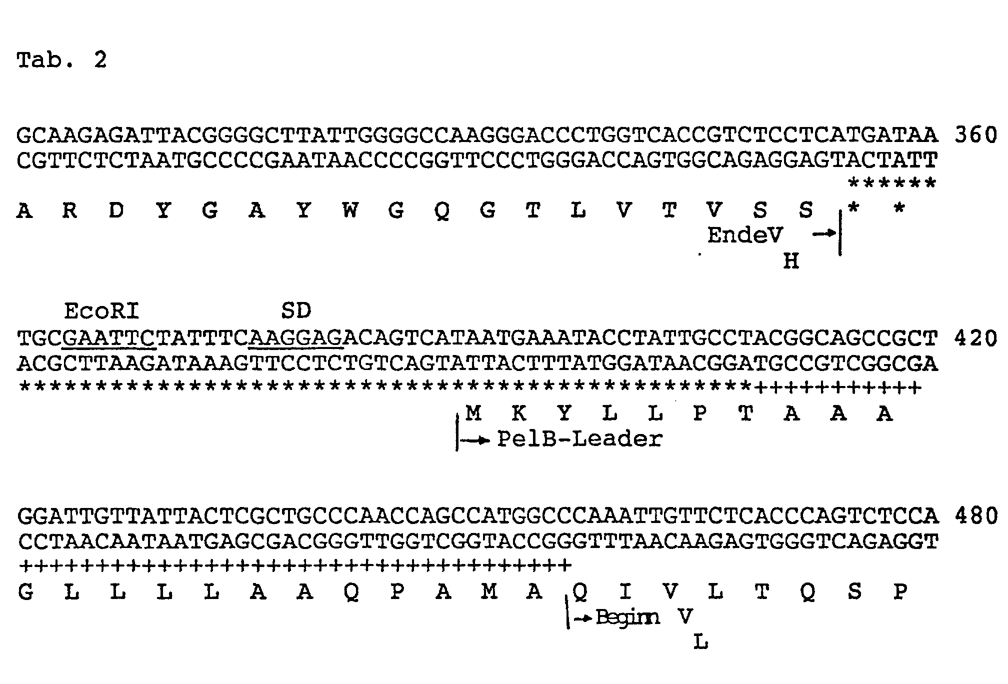

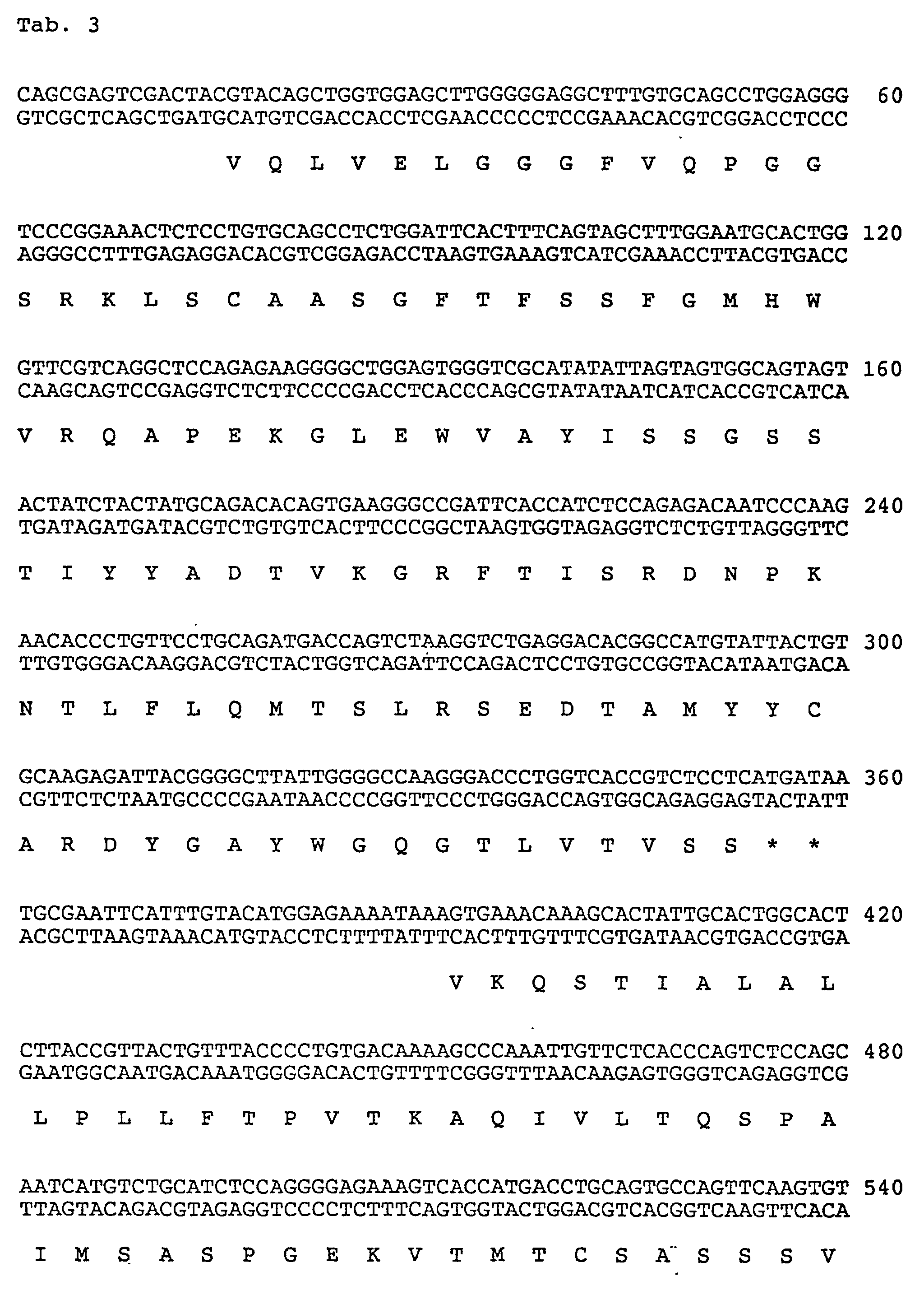

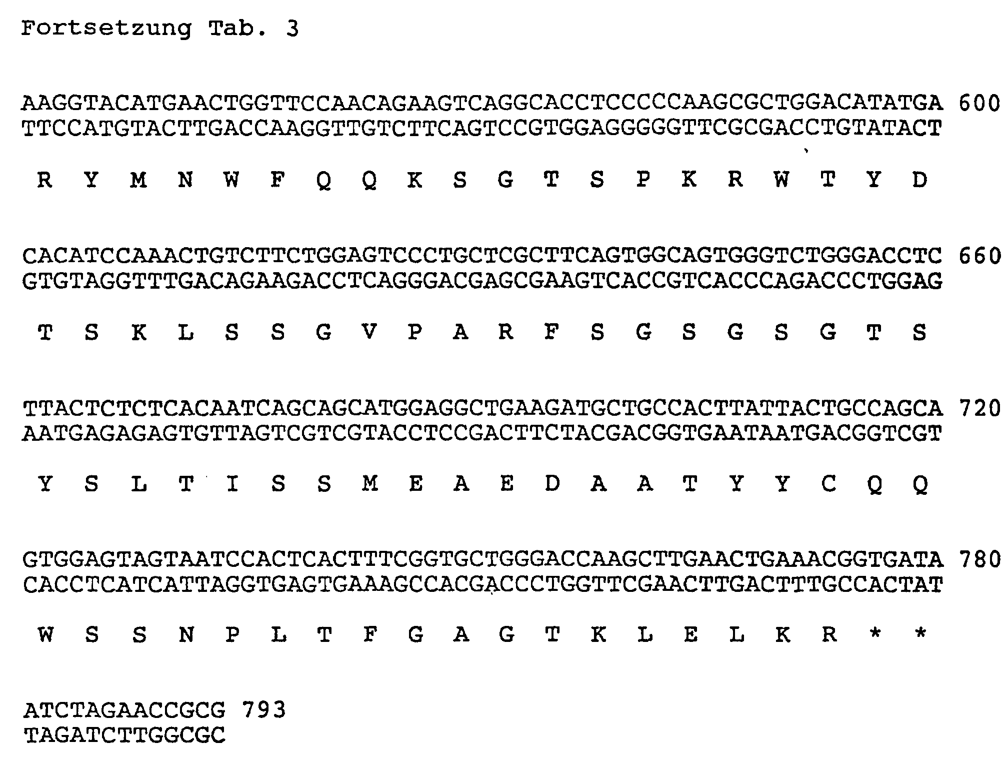

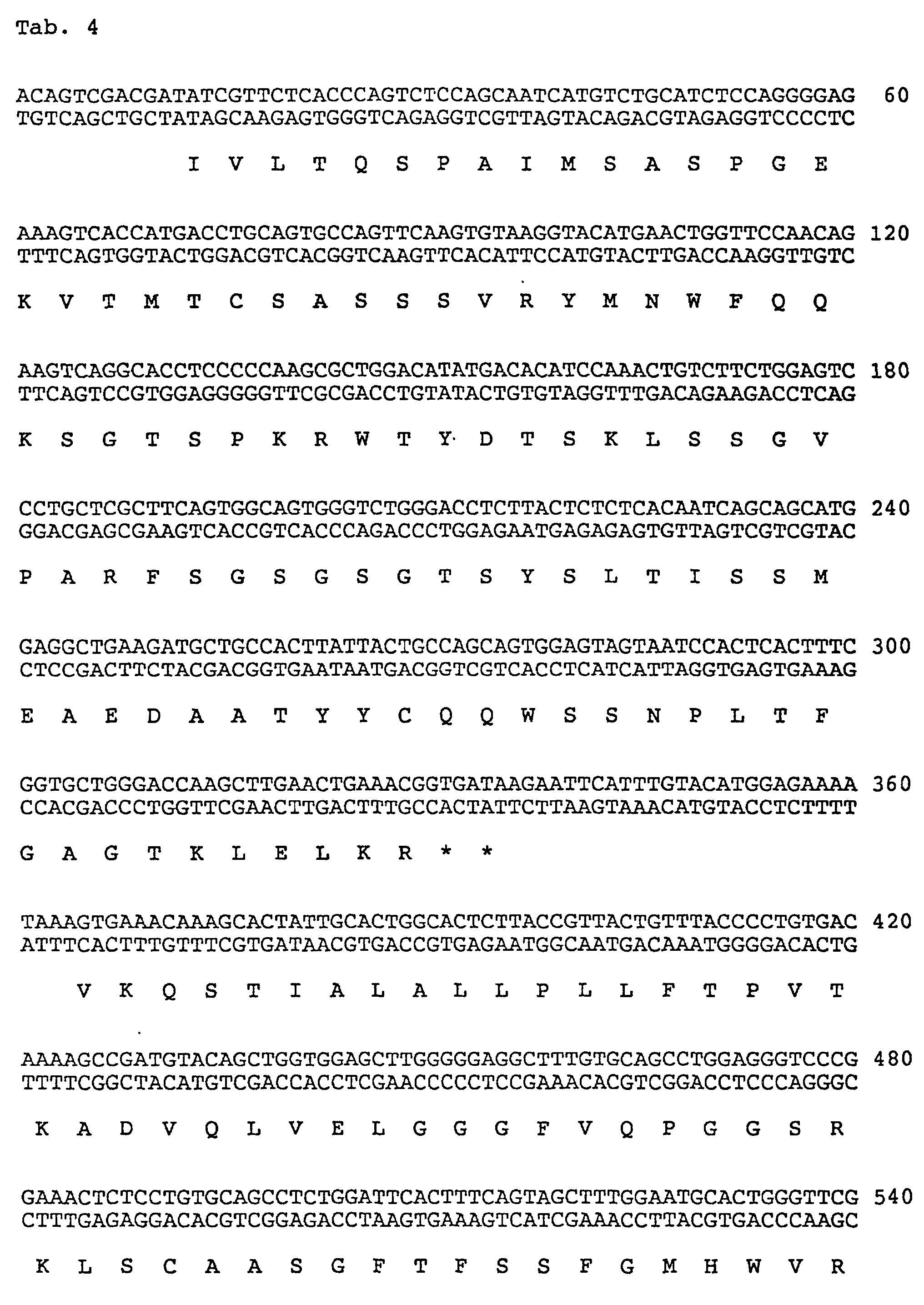

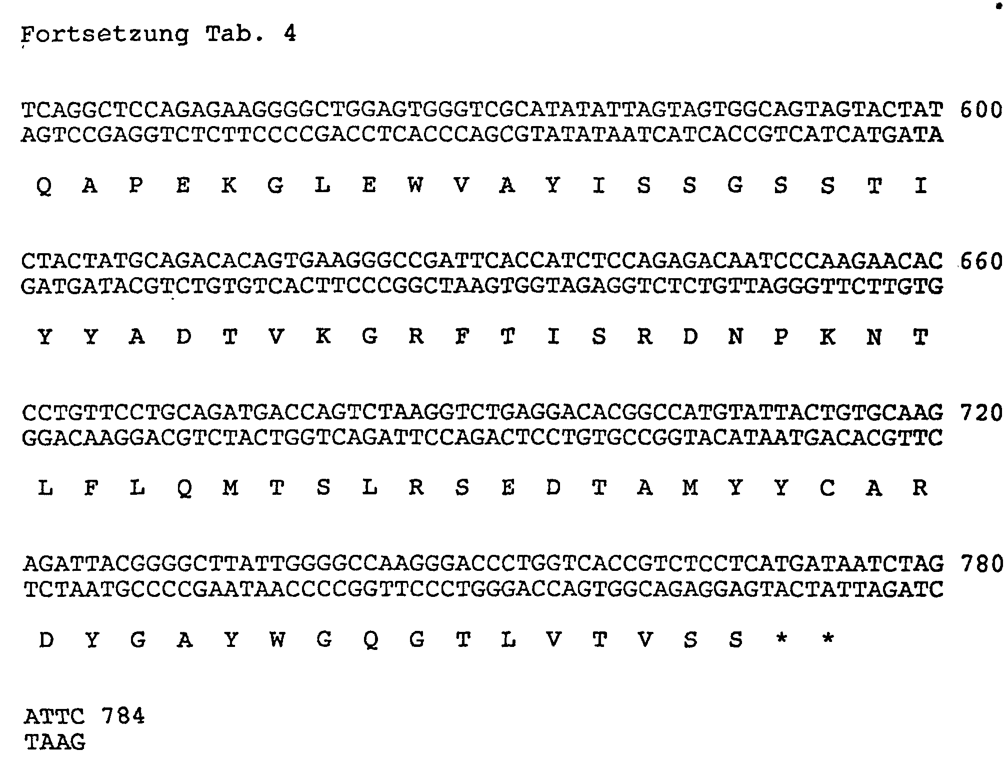

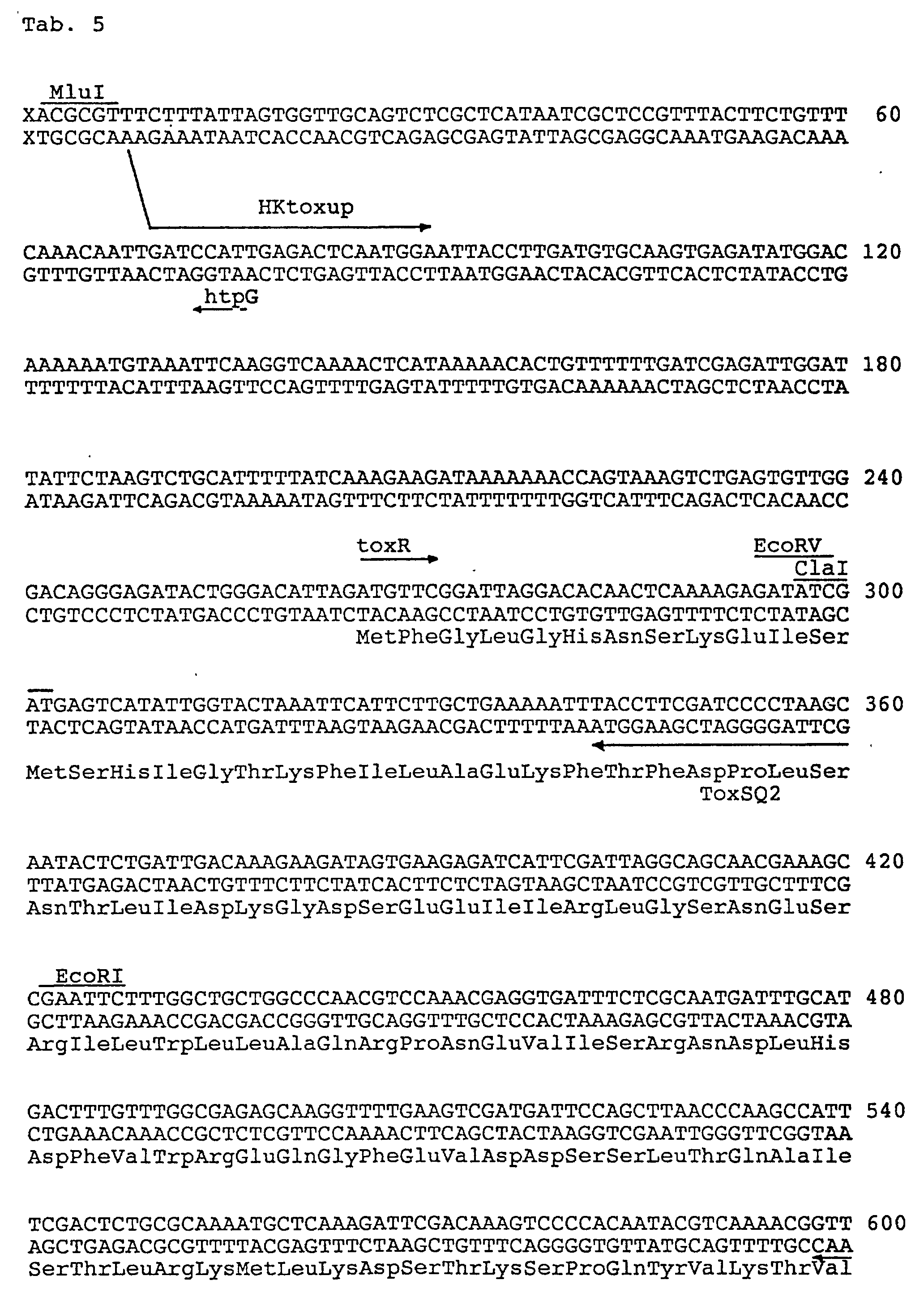

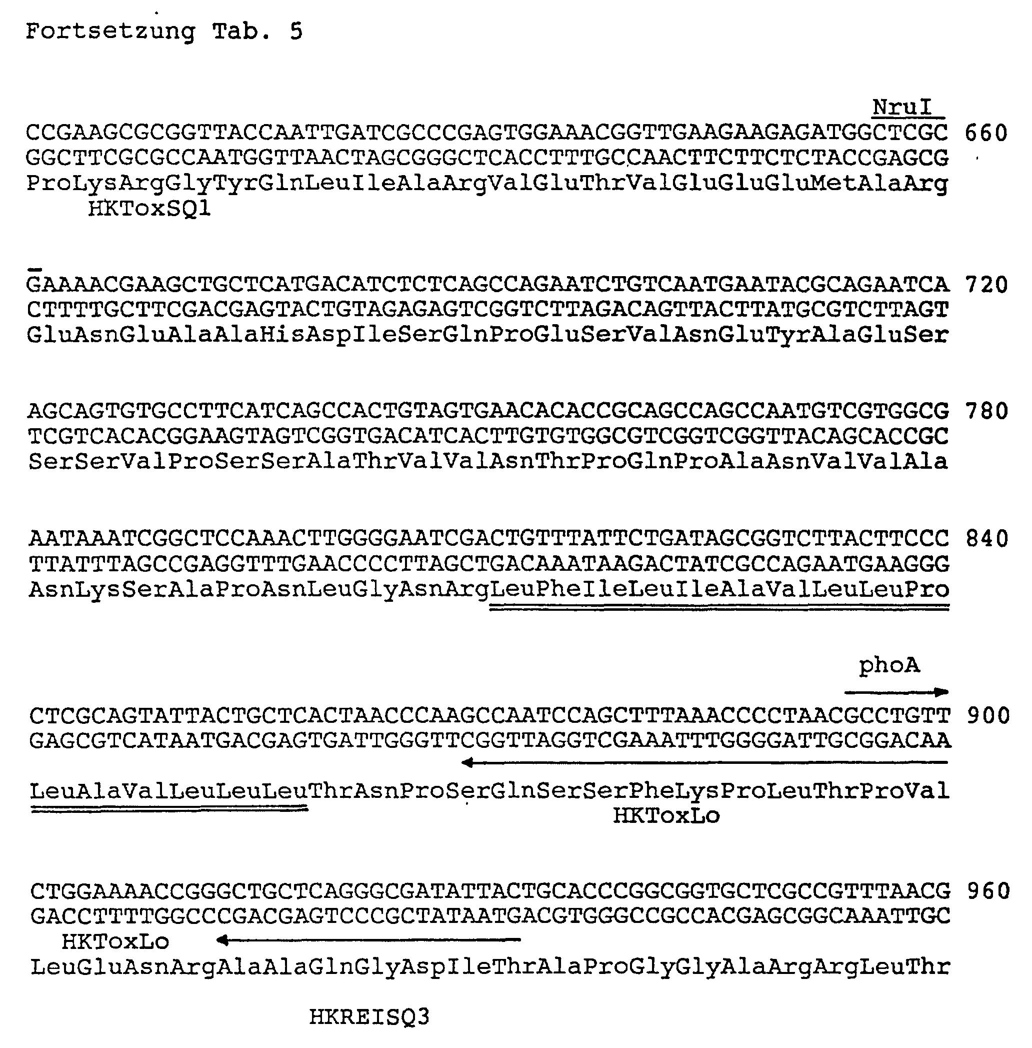

## Patentansprüche

1. Ein isoliertes Replikon, **dadurch gekennzeichnet, daß** es eine DNS-Sequenz enthält, die für ein Fusionsprotein kodiert, wobei das Fusionsprotein
a) die ToxR Regulationsdomäne, und
b) die ToxR Transmembranhelix und
c) die Immunglobulindomäne des Bence-Jones Proteins REI enthält.

## Claims

1. An isolated replicon, which contains a DNA sequence which encodes a fusion protein, with the fusion protein containing
a) the ToxR regulatory domain and
b) the ToxR transmembrane helix and
c) the immunoglobulin domain of the Bence Jones protein REI.

## Revendications

1. Réplicon isolé, **caractérisé en ce qu'**il renferme une séquence d'ADN codant pour la protéine fusionnée, la protéine fusionnée contenant
a) la région régulatrice ToxR, et
b) l'hélice transmembranaire ToxR et
c) le domaine immunoglobuline de la protéine Bence-Jones REI.
